# EUROPEAN PATENT SPECIFICATION

(11) **EP 0 948 309 B1**
(45) Date of publication and mention of the grant of the patent: **11.06.2003**
(21) Application number: 97948863.2
(22) Date of filing: 03.11.1997
(51) Int. Cl.: A61K 7/035, A61K 7/48

(54) **SPRAY-DRYED POWDER COMPRISING AT LEAST ONE PROTEIN AND ONE HYDROLYSED STARCH AND ITS USE FOR TOPICAL COMPOSITIONS**
SPRÜHGETROCKNETES PULVER FÜR TOPISCHE ZUSAMMENSETZUNGEN DAS EIN PROTEIN UND WEIZENPROTEINHYDROLYSAT ENTHÄLT
POUDRE SECHEE PAR PULVERISATION CONTENANT AU MOINS UNE PROTEINE ET UN AMIDON HYDROLYSE, ET SON UTILISATION DANS DES COMPOSITIONS TOPIQUES

(30) Priority: 06.11.1996 GB 9623026
(43) Date of publication of application: 13.10.1999
(73) Proprietor: The Boots Company PLC, Nottingham, Nottinghamshire NG2 3AA (GB)
(72) Inventor: EVISON, Jane, Nottingham NG2 3AA (GB); GALLEY, Edward, Nottingham NG2 3AA (GB); HAMILTON, Lloyd, George, Nottingham NG2 3AA (GB); BUTCHER, Kate, Elizabeth, Nottingham NG2 3AA (GB)
(74) Representative: Jones, Stephen Anthony
(86) International application number: EP9706056
(87) International publication number: WO98019652

(56) References cited:
- WO-A-94/01001
- WO-A-94/08468
- GB-A- 1 534 861
- CHEMICAL ABSTRACTS, vol. 114, no. 14, 8 April 1991 Columbus, Ohio, US; abstract no. 129150, XP002060446 & JP 02 264 721 A (TAMA BIOCHEMICAL CO. ) 29 October 1990
- CHEMICAL ABSTRACTS, vol. 123, no. 22, 27 November 1995 Columbus, Ohio, US; abstract no. 296247, XP002060447 & JP 07 206 663 A (LION CORP, JAPAN) 8 August 1995
- CHEMICAL ABSTRACTS, vol. 123, no. 12, 18 September 1995 Columbus, Ohio, US; abstract no. 152609, XP002060448 & JP 07 157 422 A (NIPPON OILS & FATS) 20 June 1995
- CHEMICAL ABSTRACTS, vol. 122, no. 26, 26 June 1995 Columbus, Ohio, US; abstract no. 322240, XP002060449 & JP 07 097 592 A (SANEIGEN EFU EFU AI KK) 11 April 1995

## Description

The invention relates to anhydrous and hydrophobic compositions for non-oral use, to methods of preparing such compositions, to methods of using such compositions. Such compositions have advantageous and unexpected properties over the prior art.

Anhydrous compositions are substantially free of free-water. Hydrophobic compositions do not substantially mix with aqueous or hydrophillic compositions. Hydrophobic compositions may comprise water if their continuous phase is substantially non-aqueous or oily (e.g. a water-in-oil emulsion). Both anhydrous and hydrophobic compositions have the disadvantage that they are incompatible with many useful ingredients which are miscible with or solvated by aqueous media. It would be advantageous to be able to formulate with a wider range of ingredients in such compositions. It is an object of the invention to solve at least some of the above problems with the prior art compositions.

The applicant has developed compositions which overcome some or all of the preceding disadvantages when formulated as anhydrous and/or hydrophobic compositions.

Spray-drying is a technique which has been used mainly in the food industry to produce powder from liquid compositions. The composition to be spray dried is atomised into small droplets, and fed into the top of a tall tower through which hot air is passed (typically at 180°C to 250°C). The more volatile components of the composition evaporate. As the droplets fall through the tower the temperature drops and they condense to produce small particles. The particles are separated and collected by a suitable method (e.g. in a cyclone).

Spray-drying typically produces fine, uniform powders of small, hollow-cored particles.

Other similar techniques to spray-drying include freeze-drying and vacuum drying. To freeze-dry a composition it is cooled until it solidifies and placed under reduced pressure to cause the most volatile ingredients in the composition to sublime. The solid residue may form a single mass which requires milling to form a fine powder. A typical freeze-dried powder comprises porous irregular shaped particles and readily hydrates. As freeze-drying does not require strong heat it is used to produce powders which comprise volatile ingredients (e.g. instant coffee granules). The drawback of powders produced by freeze-drying over spray-drying is that they are about double the cost due to the extra complexity of the freeze-drying process (e.g. equipment to operate at reduced pressures).

Vacuum drying is a similar technique to freeze-drying in which the composition remains at ambient temperature when it is under reduced pressure.

Spray-drying is the preferred technique to produce the powder of the invention although it may be possible to produce this powder by freeze-drying or vacuum-drying. The terms "spray-drying" and "spray-dried" are used herein for simplicity but the skilled person will appreciate that freeze-drying and vacuum drying can be substituted for spray-drying as appropriate.

Foodstuffs have been prepared by spray-drying but the technique has not been widely used to prepare topical compositions such as cosmetic or toiletries formulations. This is because there was little advantage perceived in spray-drying topical compositions and the specialised equipment required was expensive.

Various spray-dried anti-perspirant actives are components of known anhydrous anti-perspirant products. Such anti-perspirants are disclosed in (amongst others): US 5135741 (Chesebrough Ponds); US 5114705 (Gillette); EP 0405598 (Dow Corning); and GB 1534861 (Wicks). These spray-dried anti-perspirant powders comprise compounds such as aluminium chlorohydrate and zirconyl hydroxychloride which are highly charged.

Compounds with a high ionic charge could not be spray-dried with the water-soluble matrices used in the applicant's invention and the above prior art teaches away from this invention. However after spray-drying the powders used in the invention would be compatible with such compounds. Therefore compositions of the invention may comprise anti-perspirant active ingredients if such ingredients are not in the spray-dried powder.

JP 60-042317 (Hikiyo) describes the spray-drying of oily ingredients to form a powder for incorporation into an aqueous composition to remove the need for emulsifiers in the resultant composition.

South Korean patent application KR 92-05639 (Pacific Chemical) discloses a spray-dried material prepared from a biologically active ingredient. This ingredient is dissolved in an agar solution with a phospholipid to form, after spray-drying, a liposome of 1-500 microns which can be added to make up. The phospholipid is not a water soluble matrix as used in the powders of the present invention.

JP 61-030508 (Nippon Oils & Fats) describes an emulsion of a squalane compound coated with gum arabicum, carraginan, sodium arginate or carboxymethyl cellulose and optionally a surfactant which is spray-dried to form a powder which is used as a cosmetic substrate optionally as an anti-bacterial or anti-fungal agent.

JP 52-69187 (Suntory) describes a spray-dried powder formed from a mixture of dextrin, gum arabic or carboxymethylcellulose (CMC) with an extract of Tencha (a Chinese hydrangea). The powder may be used in foods or cosmetics and has a deodorising effect against compounds comprising S-H bonds.

DD 112351 (Neuman) discloses a spray-dried complex dermatologically active principle consisting of the proteins (e.g. whey protein), vitamins and salts contained in milk, the powder being for use in cosmetics.

Food Ingredients - Processing International (14 October 1991) discusses certain special starches as alternatives to arabic gum as a fat binder and emulsifier in foodstuffs, such starches optionally being spray dried.

Food-Prod-Des 1993 2(10), 67-68 describes use of certain gum arabic products as carriers to replace food starch for spray dried flavours. (it is not clear whether the gum is also spray dried).

None of the preceding documents suggest addition of spray-dried powder comprising a water soluble matrix to anhydrous or hydrophobic non-oral compositions.

The spray-dried powder used in the invention has unusual and surprising properties useful to a formulator of anhydrous and hydrophobic non-oral compositions especially those for topical use. The incorporation of the spray dries powder into such compositions enhances their properties.

The spray-dried powder comprises a water-soluble absorbent matrix optionally mixed with at least one active ingredient. The powder may also comprise ingredients which are incompatible with anhydrous or hydrophobic compositions when added directly. Compositions with this powder exhibit desirable properties such as improved skin-feel. Surprisingly the powder used in the invention also provides the additional unexpected advantage of synergistically enhancing the delivery of compositions to which it is added.

Therefore one aspect of this invention comprises a substantially anhydrous and/or hydrophobic composition in the form of lipsticks, soaps, deodorant sticks, sunscreen sticks, eye pencils, nail enamels or nail lacquers comprising up to 10% by weight of a spray dried powder which is a substantially water-soluble absorbent matrix containing at least one protein obtained from legumes and at least one hydrolysed starch wherein the ratio by weight of protein to hydrolysed starch is in the range 1:20 to 1:2.

Preferably the water-soluble absorbent matrix is hygroscopic and can be rapidly rehydrated.

The water-soluble absorbent matrix comprises a mixture of at least one protein from legumes and at least one hydrolysed starch in which the ratio by weight of protein to hydrolysed starch lies in the range 1:10 to 1:4.

The proteins are obtained from legumes. The protein may be a concentrate (at least about 70% protein), preferably an isolate (at least about 90% protein). Preferably, the protein is obtained from beans (e.g. soya beans and/or broad beans) and/or peas (e.g. yellow peas). Most preferably the protein comprises protein isolated with high purity (at least about 90% protein on at least about 95% dry matter) from the kernel of yellow peas, such as that available commercially from Cosucra in Belgium under the trade mark 'Pisane'®. The average amino acid content (expressed as relative proportions of mass) in the Pisane ® protein is approximately as follows: glycine 4.3; alanine 4.6; valine 5.1; leucine 8; isoleucine 4.7; serine 5.3; threonine 4; tyrosine 4; aspartic acid 12; phenylalanine 5.3; tryptophan 0.9; proline 4.5; methionine 0.93; cysteine 1; lysine 8.2; histidine 2.8; arginine 8.9; and glutamic acid 18.5.

The protein (such as highly concentrated or isolated protein fractions) may be prepared by known techniques such as wet processing, for example separation by solubilization of matrix followed by isoelectric precipitation for subsequent recovery. Other suitable known separation processes for extracting proteins comprise 'salting-out', 'hydrophobic-out' and ultrafiltration.

The hydrolysed starch preferably has a molecular weight in the range 1,000 to 100,000 and is readily soluble in water. Suitable hydrolysed starch include maltodextrin, hydrolysed potato derivative, hydrolysed corn derivative, hydrolysed maize derivative, hydrolysed oat derivative, hydrolysed tapioca derivative, cyclodextrin, dextrin (from corn, tapioca etc), pullulan and cellulose derivatives such as ethyl cellulose dispersion. A preferred hydrolysed starch is maltodextrin having a molecular weight in the range 1,000 to 10,000.

The powder used in the invention may be formed by spray-drying any suitable liquid containing the matrix. The liquid to be spray-dried may be in the form of a solution, emulsion, dispersion or suspension. The liquid preferably comprises water as a carrier material though any liquid which can be removed during spray drying can be used as the carrier material. The amount of water should be the minimum needed to ensure that the components of the liquid feed to the spray drier are intimately mixed and to ensure that the liquid can be efficiently sprayed into the spray drier. Other components which are intended to be incorporated into the spray dried powder may be dispersed, dissolved or suspended in the carrier material. Examples of such materials include oils (eg vegetable, mineral or silicone oils), emulsifiers, thickeners (eg xanthan gum or carboxymethylcellulose), moisturisers (eg 1,3-butyleneglycol), active ingredients to be more fully described hereinafter, organic sunscreens such as octylmethoxycinnamate and inorganic sunscreens such as titanium dioxide. The liquid may also comprise ingredients which are incompatible with an anhydrous or a hydrophobic composition when added directly to it, but which can be incorporated into the composition using the spray dried powder of the present invention. The liquid to be spray-dried is substantially free of any ingredient which is incompatible with the matrix. Such incompatible ingredients may comprise: species which are substantially non isoelectric with the matrix (for example species with a very high ionic charge [such as highly polyvalent ions] and/or compositions with a very low or high pH); enzymes which will attack the matrix (such as proteinases and/or amylases); and ingredients which will cause flocculation. If the mixture to be spray-dried comprises an aqueous composition preferably it has a pH of at least about 5 (more preferably between about 6 to about 7) as at a lower pH the matrix is more likely to be insoluble. If the liquid to be spray-dried comprises an aqueous carrier, the liquid may further comprise pH adjusting agents such as sodium hydroxide, aminomethyl propanol, triethanolamine, suitably in an amount of from about 0.01% to about 10% by weight of the composition. Such compositions may be buffered by means well known in the art, for example by use of buffer systems comprising succinic acid, citric acid, lactic acid, and acceptable salts thereof, phosphoric acid, mono- or di-sodium phosphate and sodium carbonate. A suitable pH may be between about 3 and about 10, preferably between about 4 and about 8, more preferably about 6. Preferably the matrix is isoelectric with the liquid to be spray-dried.

If the liquid to be spray-dried comprises an oil-in-water emulsion it may comprise from about 5% to about 60% by weight of an oil phase, from about 1% to about 20% by weight of an emulsifier or a mixture of emulsifiers and at least about 35% by weight of an aqueous phase.

Suitable oils for inclusion in the spray dried powder include vegetable oils such as wheatgerm oil, mineral oils such as paraffin oils or silicone fluids. Mixtures of oils may be used. The oils may be incorporated into the liquid to be spray dried as the oil phase of a water-in-oil emulsion. The oils may comprise 15 to 50%, preferably 20 to 40%, by weight of the spray dried powder. The inclusion of oils in the powder used in the present invention provides cosmetic and toiletries formulations which act as moisturisers. The use of the powders in the present invention containing such oils enables moisturising oils to be incorporated into compositions into which it would be difficult or impossible to incorporate such oils directly.

Once the liquid has been spray-dried the resultant powder is compatible with many ingredients which would have broken down the matrix during spray-drying.

The spray-dried powder may comprise one or more surfactants (emulsifiers). Preferably, the surfactant comprises from about 1% to about 10% by weight of the powder. Suitable surfactants (which may also act as an emulsifier if necessary) include salts of alkyl ether sulphates; alkyl betaines; alkylarnidoalkyl betaines; polyethyleneglycol carboxylates; salts of alkyl sulphates (such as ammonium lauryl sulphate); sulphosuccinates (such as disodium laureth sulphosuccinate); amphomonoacetates and diacetates (such as disodium cocoamphodiacetate); alkylpolyglucosides; alcohol sulphonates; ethoxylated fatty alcohols; ethoxylated fatty acid esters (such as ethoxylated stearates); sorbitan esters; ethoxylated sorbitan esters; silicon emulsifiers (such as silicone polyols); anionic emulsifiers (such as cetylstearyl sulphates and anionic esters of mono and diglyercides); cationic emulsifiers; fatty acid soaps (such as potassium stearate); ethoxylated mono-, di-, and tri-glycerides; phospholipids (such as lecithin); and sorbitan monooleate and mixtures thereof.

The spray-dried powder optionally contains an active ingredient. Suitable active ingredients are those which would be incompatible with anhydrous or hydrophobic compositions when added directly to such compositions. When compositions of the invention are applied topically, water in the environment (for example humidity in the atmosphere, moisture on the skin or saliva: on the lips) may re-hydrate the spray dried powder to activate any active ingredients to provide their additional properties (e.g. moisturising) to the composition.

The active ingredient comprises any ingredient which is compatible with the water-soluble absorbent matrix and which has an action on the body when administered thereto and comprises at least one suitable pharmaceutical active, cosmetic ingredient, toiletries ingredient and/or any compatible mixture thereof. The active ingredient may perform more than one function falling into one or more of the preceding categories. Preferably the active ingredient is present in an effective amount. Preferred active ingredients comprise compatible pharmaceutical, cosmetic and/or toiletries ingredients which may be medicated or unmedicated.

A pharmaceutical active ingredient comprises any pharmaceutically acceptable, physiologically active ingredient (including mixtures) which is suitable for non-oral application to a patient in need thereof. This includes all suitable physiologically active compounds, acceptable derivatives thereof (such as salts) and prodrugs (ingredients that are metabolised in vivo to give physiologically active species). The pharmaceutical active may be used to treat (by therapy and/or prophylaxis) clinical conditions (including any disorders and/or diseases) in a patient (including humans) in need thereof whether by localised application to a particular site and/or generalised systemic application.

A cosmetic active ingredient comprises any ingredient (including mixtures) which when applied will alter and/or enhance appearance. Preferred cosmetic ingredients which may be incorporated into the powders and into the compositions of the invention have pigmentary and/or moisturising properties when applied topically to normal human skin or lips. Suitable moisturisers include D-panthenol and 1,3-butylene glycol. Suitable pigments comprise various inorganic and organic pigments which are preferably powders. Suitable inorganic pigments, especially for use in lipsticks, comprise iron oxides, ultramarines and titanium dioxide. Pigments may further comprise inorganic pigments that give a pearly finish, for example bismuth oxychloride and titanium dioxide coated mica. Suitable organic pigments, especially for use in lipsticks, comprise various suitable aromatic components including monoazo, indigoid, fluoran and pyrazole dyes. Pigments are selected to be cosmetically acceptable and to comply with the laws and regulations of the territories in which they are to be used. For example in the European Union at the priority date of this application, suitable pigments are those listed in Annex IV of the Cosmetics Directive 76/768 EEC.

A toiletries active ingredient comprises any suitable ingredient (including mixtures) which is used for the purpose of personal hygiene and/or when applied, preferably topically, has a cleansing action on the body.

The spray-dried powder may also be surface-treated with suitable agents (such as aluminium stearate, amino acids, mineral oils, phospholipids, silicone oil and/or mixtures thereof) which coat the powder surface, render the particles hydrophobic in nature, prevent agglomeration and/or aid dispersion of powder within a composition.

Lipsticks, soaps, deodorant sticks, sunscreen sticks or eye pencils,according to the present invention contain up to 10% preferably 0.5 to 5% more preferably 1 to 3% of the spray dried powder. Nail enamels or lacquers may contain up to 5% preferably 0.1 to 3%, more preferably 0.25 to 2% of the spray dried powder.

The applicant has surprisingly discovered that the powder used in the invention can act synergistically with other ingredients in such compositions to enhance the delivery, preferably topically delivery, of the composition and/or any active ingredients therein. This improved delivery is a unexpected further advantage of compositions of the invention. For example when the composition comprises a pigmented lipstick there is enhanced delivery of colour to the lips. Data to illustrate the synergistically enhanced topical delivery of compositions of the invention are provided in the Examples.

Compositions which deliver more composition per application (e.g. topically when applied to the skin) have an enhanced effect. Alternatively reduced quantities of ingredients are require to achieve the same effect Thus money may be saved on raw materials, undesirable effects of any ingredient may be reduced and less potent ingredients can be substituted. If less active ingredient can be used this also allows compositions to be applied to sensitive sites on the body.

Pharmaceutical compositions comprise the spray dried powder containing a therapeutically and/or prophylactically effective amount of at least one pharmaceutical active ingredient with a pharmaceutically acceptable and compatible diluent or carrier.

Nail lacquers include nail varnishes, top coats, base coats, nail hardeners and ridge fillers.

Lipsticks may be medicated or unmedicated and comprise lip salves; lip balms; lip glosses; solid compositions to treat sore, cracked and/or split lips; and/or pigmented sticks which colour the lips. The choice of colour or shade is a very important consideration for the consumer when purchasing any pigmented cosmetic, especially a lipstick. Consumers demand a wide choice of colours and shades. However more intense shades and colours can be difficult to produce due to the difficulty of formulating compositions with high concentrations of pigment. This is because homogeneous dispersions of large amounts of pigment in an oil can be unstable. Also large concentrations of pigment may have a greater potential for allergic reaction on the skin. The invention provides lipsticks which can deliver more pigment to the skin to provide new, more intense shades of colour. Alternatively less pigment can be used to produce a known colour shade which both saves money and reduces the possibility of unwanted bleeding of colour away from the site of application. This is especially useful for lipsticks as pigment can be more readily retained on the lips reducing unsightly spread of colour onto the skin adjacent to the lips. Lipsticks may further comprise one or more cosmetically acceptable additional ingredients suitable for lipsticks selected from: gloss modifiers, texture modifiers, moisturising agents, soothing agents, conditioners, vitamins and/or sunscreens.

Sunscreens encompass tanning lotions, sunscreens and sunblockers which are intended for use on the body to provide protection against the sun's rays or other UV sources. Sunscreen agents which may be incorporated into the compositions of the present invention comprise organic chemical agents which act to absorb incident UV radiation and/or inorganic sunscreen agents which act to reflect incident UV radiation. Examples of suitable sunscreening agents comprise one or more of the following or any suitable mixtures thereof: p-aminobenzoic acids, esters and derivatives (e.g. octyl-p-dimethylaminobenzoate); methoxycinnamate esters (e.g. 2-ethylhexyl-p-methoxycinnamate, or 2-ethoxyethyl-p-methoxycinnamate); benzophenones (for example oxybenzone); dibenzoylmethanes; salicylate esters; metal oxide sunscreens (such as TiO₂, preferably of having mean primary particle size between 1 to 100 nm, or ZnO preferably having a mean primary particle size between 30 and 300nm or any mixture thereof). Preferably any sunscreening agent is present in an amount from about 0.1% to about 25%, more preferably 0.5% to 15% by weight of the composition.

The percentage of pigment used in a cosmetic composition will depend upon the type of cosmetic being formulated. Lipsticks and similar cosmetics will contain higher percentages of pigment than other cosmetics. Lipsticks and other highly coloured compositions may contain up to 15% w/w of pigment by weight of the composition.

Cosmetic compositions of the invention (for example lipsticks) may comprise one or more oil components, one or more wax components and/or one or more additional powder components. Preferably the cosmetic compositions contains from about 30% to about 85% w/w of the oil component; from about 1% to about 40% w/w of the wax component; and from about 1% to about 40% w/w of the additional powder component.

The oil component may comprise one or more cosmetically acceptable oils, silicones, fats and/or any mixture thereof. Suitable cosmetically acceptable oils comprise natural oils (for example plant oils [such as almond oil, vegetable oil and castor oil]), synthetic esters (for example pentaerythrityl tetracaprylate and pentaerythrityl tetraisostearate), fatty oils (for example triglyceride esters), fatty alcohols (for example oleyl alcohol and octyldodecanol) and mineral oils (for example liquid paraffin and C₁₁₋₁₂ isoparaffin). Suitable cosmetically acceptable silicones may comprise volatile silicones (for example volatile linear polydimethylsiloxanes) and cyclomethicone and non-volatile silicone oils (for example phenyldimethicone). Suitable cosmetically acceptable fats may comprise natural fats (for example animal fats [such as wool fat], vegetable fats [such as triglyceride esters] and mineral fats [such as white soft paraffin]) and synthetic fats (for example bisdiglyceryl caprylate / caprate / isostearate stearate / hydroxystearate adipate). Preferably the oil component is present in an amount from about 50% to about 80% w/w. Optionally the oil component may further comprise soothing agents (for example alpha bisabolbol, a plant extract, such as camomile extract and/or moisturising agents (for example an alkylene glycol [such as butylene glycol]).

The wax component preferably has a melting point in the range from about 30 °C to about 120 °C. Suitably the wax component comprises one or more cosmetically acceptable waxes, wax-like materials and/or any mixture thereof. Suitable waxes comprise natural waxes (for example plant waxes [such as candelilla wax, carnauba wax, castor wax and illupe butter], animal waxes [such as beeswax, lanolin wax, lanolin derivative waxes and white wax] and mineral waxes [such as ozokerite wax, montan wax and paraffin waxes {e.g. microcrystalline wax, okerin wax and paraffin}]), synthetic waxes (for example hydrogenated castor oil and cetyl alcohol) and silicone waxes (for example C₂₄₋₂₈ alkyl methicone). Preferably the wax component is present in a cosmetic composition in an amount from about 5% to about 40% w/w, more preferably from about 10% to about 30% w/w.

Preferably, the additional powder component is present in an amount from about 2% to about 30% w/w. If the cosmetic composition is a lipstick, the additional powder component may comprise a dry, particulate material having a mean primary particle size from about 0.02 microns to about 80 microns. Particle size is measured along the longest axis of the particle. The additional powder component may comprise pigmentary powder and/or non-pigmentary powder. Suitable pigmentary powders comprise various inorganic and organic pigments such as those described herein. Suitable non-pigmentary powders comprise acrylate polymers, alumina, aluminium silicate, bentonite, boron nitride, calcium carbonate, calcium silicate, cellulose, corn starch, kaolin, magnesium aluminium silicate, magnesium carbonate, mica, micronised PTFE, nut shell powder (such as walnut shell powder), nylon, polyethylene, polymethyl methacrylate beads, silica, silk powder, tin oxide, zinc myristate and any mixtures thereof.

Preferred topical cosmetic compositions of the invention are lipsticks further comprising a spray-dried powder with a suitable pigment and/or moisturiser as an active ingredient.

Toiletries compositions comprise the spray dried powder containing an effective amount of at least one toiletries ingredient with a topically acceptable and compatible diluent or carrier. Preferred toiletries compositions include soaps.

Topical compositions of the invention may also comprise ionic or non-ionic surface active agents to promote greater effectiveness (e.g. improved therapeutic and/or prophylactic activity) in the compositions if applied topically. The surface active agents may also comprise emulsifying ingredients and/or surfactants even if the compositions are not emulsions.

Topical compositions of the invention may additionally comprise further components well known to those skilled in the art and which are suitable and directly compatible with an anhydrous and/or hydrophobic composition. Such ingredients may comprise any of the optional ingredients as described herein and/or any suitable and compatible mixtures thereof.

Compositions of the invention that have a high lipid solubility may be suitable for use in so-called depot formulations which provide a source of active ingredient located within the body (for example by intra-muscular injection). Depot formulations may comprise active ingredients in a pharmaceutically acceptable oil.

In some formulations it may be beneficial to use powders and/or compositions of the invention in the form of particles of very small size, for example as obtained by fluid energy milling. Compositions of the invention which are solid at ambient temperature may be compressed in the form of a stick and/or incorporated into anhydrous sticks (e.g. hot wax sticks) or extrudates.

Acceptable diluents and/or carriers suitable for compositions of the invention are well known to a skilled formulator. The excipients used in the preparation of such compositions are the excipients known in the formulator's art.

Solid compositions of the invention may be prepared by mixing the spray-dried powder with one or more of the following ingredients which are acceptable in a solid anhydrous and/or hydrophobic composition: inert diluents, lubricating agents, binders and/or any mixtures thereof. It will be appreciated by those skilled in the art that a particular ingredient may perform more than one function (for example maize starch may act as a diluent, binder and/or disintegrating agent).

Inert diluents may comprise sugars (for example lactose, dextrin, sucrose, mannitol, powdered sugar and/or mixtures thereof), celluloses (for example microcrystalline cellulose), starches (for example maize starch, other pharmaceutical grade starch and/or mixtures thereof), clays (for example kaolin and/or hectorite), calcium phosphate, calcium sulphate and/or mixtures thereof.

Lubricating agents may comprise magnesium stearate, calcium stearate, stearic acid, talc, paraffin sulphate, boric acid, sodium benzoate, sodium acetate, sodium chloride, leucine, polyethylene glycol and/or mixtures thereof.

Binders may comprise starches (for example maize starch), gelatin, sugars (for example sucrose, molasses, lactose and/or mixtures thereof) and/or natural and/or synthetic gums (for example acacia, sodium alginate, extract of Irish moss, celluloses [such as carboxymethylcellulose, hydroxypropylmethyl cellulose, methylcellulose, ethylcellulose, microcrystalline cellulose and/or mixtures thereof], polyethylene glycol, waxes, polyvinylpyrrolidone and/or mixtures thereof).

Solid compositions of the invention may further comprise one or more of the following acceptable ingredients and/or mixtures thereof that are known to aid production of solid compositions:-
agents to aid the flow of ingredients (for example talc and/or colloidal silicon dioxide);
compression agents to increase the strength of a solid composition (for example sorbitol and/or lactose); and/or
ionic and/or non-ionic surface active agents (for example sodium lauryl sulphate) to promote an even dispersion of ingredients within a solid composition and prevent grit forming at the surface.

Preferably solid compositions of the invention are shaped to be more convenient for general use, for example in stick form.

Other components that may be appropriately added to compositions of the invention are known to those skilled in the art. Such ingredients may comprise at least one of the following and any suitable and compatible mixtures thereof (some ingredients may perform more than one function):
preservatives such as 2-bromo-2-nitropropane-1,3-diol (bronopol, which is available commercially under the trade name Myacide), benzyl alcohol, diazolidinyl urea, imidazolidinyl urea, methyl paraben, phenoxy ethanol, polyhexamethylenebiguanide hydro-chloride, isothiazolone, sodium dehydroacetate, propyl paraben, sodium methyl paraben, sodium propyl paraben, methyl p-hydroxybenzoate, propyl p-hydroxy benzoate and/or sorbic acid, suitably in an amount of from about 0.01% to about 1% by weight of the composition;
sequestering agents such as ethylenediamine tetraacetic acid and/or salts thereof, suitably in an amount of from about 0.005% to about 0.5% by weight of the composition;
vitamins, such as biotin, suitably in an amount of from about 0.01% to about 1.0% by weight of the composition;
pearlising agents such as stearic monoethanolamide, suitably in an amount of from about 0.01% to about 10% by weight of the composition.
emollients such as isopropyl myristate and/or a triglyceride of a fatty acid (e.g. lauric triglyceride and/or capric / caprylic triglyceride);
humectants such as glycerin and/or 1,3-butylene-glycol;
antioxidants such as vitamin C, d,l-α-tocopheryl acetate, butylated hydroxytoluene, parabens, butylated hydroxy toluene, butylated hydroxyannisole, propyl-p-hydroxybenzoate and/or other suitable preservatives;
emulsion stabilisers, such as suitable salts, for example sodium chloride, sodium citrate and/or magnesium sulphate;
film formers to assist spreading on the surface of the skin such as alkylated polyvinyl-pyrrolidone;
perfumes suitably in an amount of from about 0.01% to about 2% by weight of the composition;
colourings, such as water soluble dyes such as tartrazine, suitably in an amount of from about a trace amount (such as 1 x 10⁻⁵ %) to about 0.1% by weight of the composition; and
other suitable agents such as thickening agents, gelling agents, gloss modifiers, texture modifiers, soothing agents, conditioners, moisturisers and sunscreen agents.

A further aspect of the invention comprises a method of preparing an anhydrous and/or hydrophobic composition for non-oral application comprising the steps of:
(a) spray drying a liquid to form a powder, the liquid comprising water-absorbent matrix and optionally at least one active ingredient which is incompatible with an anhydrous and/or hydrophobic composition when added directly; and
(b) mixing in a suitable manner the resulting powder with an acceptable diluent or carrier to form an anhydrous and/or hydrophobic composition suitable for administration by a non-oral route.

Further steps in the method of the invention are known to those skilled in the art. For example the powder may be brought into association with suitable inert diluents, carriers and/or any other optional ingredients (for example those described herein). The ingredients in the composition may be uniformly and/or intimately admixed and the resultant composition may be shaped (for example by compressing and/or moulding). If the composition contains large amounts of excipients in relation to the spray-dried powder, repeated conventional mixing operations may be required to distribute the powder evenly and/or homogenously throughout the composition.

The invention will be understood with reference to the non-limiting Examples described below.

With the exception of the spray-dried powder of the invention (as described in Examples A, B and C), the other ingredients used in the following Examples are largely conventional. In some of these Examples only the general type of each ingredient has been indicated as the specific ingredient can be any suitable conventional ingredient of the specified general type (for example any of those described above). These would be well known to a skilled formulator. It is not believed that the choice of a specific conventional ingredient would significantly affect the advantageous properties of these Examples as far as they relate to the invention.

### Example A

### (Spray-dried powder)

| *Ingredient* | *Amount (% w*/*w)* |
|---|---|
| Vegetable glycerine | 1.73 |
| 1,3-Butylene glycol | 3.46 |
| Keltrol (thickener) | 0.34 |
| Sequestrene NA4 (powder) | 0.05 |
| Monostearin NSE (edible oil) | 2.88 |
| Polawax GP 200 (vegetable grade oil) | 4.03 |
| Cetyl alcohol (oil) | 1.85 |
| Hard paraffin (oil) | 2.88 |
| White soft paraffin (oil) | 2.88 |
| Light liquid paraffin (oil) | 4.03 |
| Silicone fluid F111/100 (oil) | 1.15 |
| Cetiol SN (oil) | 5.76 |
| Light colour wheat-germ oil | 0.34 |
| Pisane ® (protein isolate from yellow peas) | 6.71 |
| Maltodextrin (powder) | 58.94 |
| D,L-α-Tocopheryl acetate (oil) | 0.12 |
| D-Panthenol (moisturiser) | 0.58 |
| Oil of orchids | 0.28 |
| Lactic acid | 0.03 |
| Sodium lactate (60% aq. solution) | 0.07 |

A powder of Sequestrene NA4 and Pisane ® was dispersed in water at 70 - 75 °C with a Silverson high shear mixer. The mass ratio of powder to water was 1:1.67 respectively. The vegetable glycerine, butylene glycol and Keltrol were mixed together to from a slurry which was then added to the Pisane ® suspension. The oils were melted together at 70 - 75 °C and the aqueous slurry was added to this melt to form an emulsion. The maltrodextrin powder was mixed into to the emulsion with a Silverson high shear mixer. The panthenol, lactic acid and sodium lactate were added to the emulsion which was then homogenised with a Monton-Gaulin homogeniser at a pressure of 150 bar and at 75 °C. The emulsion was fed into a spray-dryer at an input temperature of 160°C and an output temperature of 82 °C to produce a powder which was collected from the bottom of the cyclone of the spray-dryer.

### Example B

### (Spray-dried powder)

| *Ingredient* | *Amount (% w*/*w)* |
|---|---|
| Sequestrene NA4 (powder) | 0.05 |
| Polawax GP 200 (vegetable grade oil) | 4.95 |
| Cithrol GMS (emulsifier) | 6.60 |
| Cetyl alcohol (oil) | 3.30 |
| Light liquid paraffin (oil) | 16.49 |
| Silicone fluid (oil) | 0.82 |
| Pisane ® (protein isolate from yellow peas) | 12.55 |
| Maltodextrin (powder) | 55.10 |

A powder of Sequestrene NA4 and Pisane ® was dispersed in water at 70 - 75 °C with a Silverson high shear mixer. The mass ratio of powder to water was 1:1.67 respectively. The oils were melted together at 70 - 75 °C and this oily melt was added to the aqueous mixture to form an emulsion. The maltrodextrin powder was mixed into to the emulsion with a Silverson high shear mixer. The emulsion was then homogenised with a Monton-Gaulin homogeniser at a pressure of 150 bar and at 80 °C. The emulsion was fed into a spray-dryer at an input temperature of 180 °C and an output temperature of 85 °C to produce a powder which was collected from the bottom of the cyclone of the spray-dryer.

### Example C

### (Spray-dried powder)

| *Ingredient* | *Amount (% w*/*w)* |
|---|---|
| 1,3-Butylene glycol (moisturiser) | 3.87 |
| Polawax GP 200 (vegetable grade oil) | 4.83 |
| White soft paraffin (oil) | 12.36 |
| Silicone fluid F111/100 (oil) | 1.47 |
| Cetyl alcohol (oil) | 2.22 |
| Hard paraffin (oil) | 4.15 |
| Light liquid paraffin (oil) | 14.48 |
| Monostearin NSE (edible oil) | 3.48 |
| Carboxymethyl cellulose gum (thickener) | 0.40 |
| Titanium dioxide sunscreen | 2.76 |
| Pisane ® (protein isolate from yellow peas) | 10.00 |
| Maltodextrin (powder) | 37.22 |

The Pisane ® and the titanium dioxide were dispersed in water at 70 - 75 °C with a Silverson high shear mixer to from a suspension. The mass ratio of powder to water was 1:1.67 respectively. The butylene glycol and cellulose gum were mixed together to from a slurry which was then added to the aqueous suspension. The oils were melted together at 70 - 75 °C and the oily melt was added to the aqueous suspension. This mixture was then mixed with a Silverson high shear mixer. The maltrodextrin powder was added to the mixture using a Silverson high shear mixer. The mixture was homogenised with a Monton-Gaulin homogeniser at a pressure of 150 bar and at 80°C. The homogenised mixture was fed into a spray-dryer with an input temperature of 160°C and an output temperature of 80°C to produce a powder which was collected from the bottom of the cyclone of the spray-dryer.

### Example 1

### (Lipstick with a spray-dried moisturising powder)

| *Ingredient* | *Amount (% w*/*w)* |
|---|---|
| Plant wax | 6.26 |
| Mineral wax | 9.00 |
| Fatty alcohol | 22.87 |
| Synthetic fat | 10.00 |
| Preservative | 0.10 |
| Anti-oxidant | 0.03 |
| 25 % Pigment in castor oil paste | 34.99 |
| Spray-dried moisturising powder (Ex. C) in oil | 4.00 |
| Butylene glycol | 1.50 |
| Castor oil | 11.25 |

The spray-dried moisturising powder was prepared as described in Example C and mixed into a castor oil base at a respective mass ratio of 1:3 powder to oil.

The pigment, spray-powder, butylene glycol and castor oil were mixed together to form a uniform paste. The remaining ingredients were added and the mixture was then melted together at 78 °C and poured into moulds to form a product.

A comparative example, Example X, was prepared as above omitting the spray-dried powder of Example C.

To the volar aspect of the forearm of forty volunteers, two sites were delineated, one to which the lipstick of Example 1 was applied and the other to which

Example X was applied as a control. Each lipstick was weighed before and after application to determine the mass of lipstick that had been applied to the skin. The mean amount of Example X applied to the skin of each subject was approximately 5.90 mg. The mean amount of Example 1 applied to the skin of each subject was approximately 7.24 mg. This represents a mean increase of approximately 22.7 % in the mass of lipstick delivered to the skin per subject for Example 1 over the control (Example X). The data are statistically significant.

Thus Example 1, which comprises the spray-powder of Example C, exhibits improved topical delivery of lipstick compared to the formulation without the powder.

### Example 2

### (Shaving soap with spray-dried moisturising powder)

| *Ingredient* | *Amount (% w*/*w)* |
|---|---|
| Shaving soap noodles | to 100 |
| Pigmentary titanium dioxide | 0.10 |
| Optical brightener | 0.01 |
| Perfume | 0.99 |
| Spray-dried moisturising powder (Ex. A) | 10.00 |

The spray-dried moisturising powder was prepared as described in Example A. The above ingredients were mixed together thoroughly and compressed into a moulds to form a soap with moisturising properties.

### Example 3

### (Deodorant stick with spray-dried moisturising powder)

| *Ingredient* | *Amount (% w*/*w)* |
|---|---|
| Stearyl alcohol vegetable grade | 13.13 |
| Hydrogenated castor oil | 5.05 |
| Cyclomethicone | 50.50 |
| Aluminium chlorohydrate | 20.20 |
| Silica thickener | 1.01 |
| Sterilised Mistron Galaxy Talc | 9.09 |
| Mixture of glyceryl stearate and PEG-100 stearate | 1.01 |
| Butylated hydroxy toulene | 0.01 |
| Oily herbal extracts | 0.20 |
| Perfume | 0.50 |
| Spray-dried moisturising powder (Ex. B) | 1.50 |

The spray-dried moisturising powder was prepared as described in Example B.

The stearate mixture, castor oil and stearyl alcohol were melted together. In a separate container the aluminium chlorohydrate and silica thickener were added to the cyclomethicone using a Silverson high shear mixer. The two mixtures were then mixed together and heated to 75 - 80 °C. The talc and butylated hydroxy toulene were added to the resultant mixture whilst it was being stirred. The mixture was stirred with a Silverson high shear mixer to ensure it was homogenous and then cooled to 75 °C whilst being stirred. The herbal extract and perfume were added and the mixture was compressed. Finally the composition was poured into suitable packaging and allowed to cool to ambient temperature to form a deodorant with moisturising properties.

### Example 4

### (Anti-perspirant stick with spray-dried moisturising powder)

| *Ingredient* | *Amount (% w*/*w)* |
|---|---|
| Cyclomethicone | 52.00 |
| Aluminium chlorohydrate | 17.00 |
| Silica thickener | 1.00 |
| Stearyl alcohol vegetable grade | 13.00 |
| Hydrogenated castor oil | 5.00 |
| Perfume | 0.50 |
| Butylated hydroxy toulene | 0.01 |
| Sterilised Mistron Galaxy Talc | 11.00 |
| Spray-dried moisturising powder (Ex. B) | 1.50 |

This Example was prepared in a similar manner to Example 3.

### Example 5

### (Sunstick of SPF 20 with 1.5 % spray-dried moisturising powder)

| *Ingredient* | *Amount (% w*/*w)* |
|---|---|
| Zinc oxide sunscreen in a synthetic ester paste | 38.57 |
| Titanium dioxide sunscreen (coated with Al stearate) in a plant oil paste | 13.33 |
| Mineral wax | 13.03 |
| Plant wax | 5.52 |
| Plant oil | 11.89 |
| Synthetic fat | 6.59 |
| Synthetic ester | 11.22 |
| Alpha bisabolbol (soothing agent) | 0.12 |
| Spray-dried moisturising powder (Ex. B) | 1.50 |

The spray-dried moisturising powder was prepared as described in Example B.

The above ingredients were melted together and then mixed with a Silverson high shear mixer to obtain a homogenous mixture. The mixture was poured into moulds to form a sunstick with moisturising properties.

### Example 6

### (Sunstick for sensitive skin of SPF 25 with 1 % spray-dried moisturising powder)

| *Ingredient* | *Amount (% w*/*w)* |
|---|---|
| Titanium dioxide sunscreen (coated with Al stearate) in a plant oil paste | 21.70 |
| Titanium dioxide sunscreen in a plant oil paste | 10.00 |
| Octyl methoxycinnamate (organic sunscreen) | 5.00 |
| Butyl methyldibenzoylmethane (organic sunscreen) | 2.50 |
| Mineral wax | 14.40 |
| Plant wax | 5.66 |
| Synthetic ester | 32.98 |
| Synthetic fat | 6.76 |
| Spray-dried moisturising powder (Ex. C) | 1.00 |

The spray-dried moisturising powder was prepared as described in Example C.

All the ingredients were mixed together apart from the organic sunscreens. The mixture was then heated until it melted and was mixed with a Silverson high shear mixer. The remaining ingredients were added to this mixture which was heated and then poured into moulds to form a sunstick with moisturising properties.

### Example 7

### (Sunstick for sensitive skin of SPF 25 with 1.5% spray-dried moisturising powder)

| *Ingredient* | *Amount (% w*/*w)* |
|---|---|
| Titanium dioxide sunscreen (coated with Al stearate) in a plant oil paste | 21.70 |
| Titanium dioxide sunscreen in a plant oil paste | 10.00 |
| Octylmethoxycinnamate (organic sunscreen) | 5.00 |
| Butylmethyldibenzoylmethane (organic sunscreen) | 2.50 |
| Mineral wax | 14.4 |
| Plant wax | 5.66 |
| Synthetic ester | 32.98 |
| Synthetic fat | 6.76 |
| Spray-dried moisturising powder (Ex. C) | 1.00 |

This Example was prepared in a similar manner to Example 6.

### Example 8

### (Sunstick for sensitive skin of SPF 25 with 2 % spray-dried moisturising powder)

| *Ingredient* | *Amount (% w*/*w)* |
|---|---|
| Titanium dioxide sunscreen (coated with Al stearate) in a plant oil paste | 21.70 |
| Titanium dioxide sunscreen in a plant oil paste | 10.00 |
| Octylmethoxycinnamate (organic sunscreen) | 5.00 |
| Butylmethyldibenzoylmethane (organic sunscreen) | 2.50 |
| Mineral wax | 14.17 |
| Plant wax | 5.56 |
| Synthetic ester | 32.43 |
| Synthetic fat | 6.64 |
| Spray-dried moisturising powder (Ex. C) | 2.00 |

This Example was prepared in a similar manner to Example 6.

### Example 9

### (Sunstick for sensitive skin of SPF 25 with 5 % spray-dried moisturising powder)

| *Ingredient* | *Amount (% w*/*w)* |
|---|---|
| Titanium dioxide sunscreen (coated with Al stearate) in a plant oil paste | 21.70 |
| Titanium dioxide sunscreen in a plant oil paste | 10.00 |
| Octyl methoxycinnamate (organic sunscreen) | 5.00 |
| Butylmethyldibenzoylmethane (organic sunscreen) | 2.50 |
| Mineral wax | 13.52 |
| Plant wax | 5.23 |
| Synthetic ester | 30.77 |
| Synthetic fat | 6.31 |
| Spray-dried moisturising powder (Ex. C) | 5.00 |

This Example was prepared in a similar manner to Example 6.

### Example 10

### (Lipstick with a spray-dried powder)

| *Ingredient* | *Amount (% w*/*w)* |
|---|---|
| Wax | 22.46 |
| Castor oil | 0.66 |
| Synthetic fat | 11.74 |
| Silicone fluid (oil) | 40.96 |
| Fatty alcohol | 0.71 |
| 1,3 Butylene glycol | 0.99 |
| Pigment in oil paste | 13.45 |
| Pearlising agent | 4.56 |
| Rosehip oil | 0.50 |
| d,l-α-Tocopheryl acetate | 0.99 |
| Octyl methoxycinnamate (organic sunscreen) | 0.99 |
| Butyl methyl dibenzoyl methane (organic sunscreen) | 0.50 |
| Polyquaternium 37 (cationic resin) | 0.50 |
| Spray-dried moisturising powder (Ex. B) in oil | 1.00 |

The spray-dried powder was prepared as described in Example B and mixed into a castor oil base at a respective mass ratio of 1:3 powder to oil.

The pigment, spray-powder, butylene glycol and castor oil were mixed together to form a uniform paste. The remaining ingredients were added and the mixture was then melted together at 78 °C and poured into moulds to form a product.

A comparative example, Example Y, was prepared as above omitting the spray-dried powder ingredient.

To the volar aspect of the forearm of forty volunteers, two sites were delineated, one to which the lipstick of Example 10 was applied and the other to which the Example Y was applied as a control. Each lipstick was weighed before and after application to determine the mass of lipstick that had been applied to the skin. The mean amount of Example Y applied to the skin of each subject was approximately 0.98 mg. The mean amount of Example 10 applied to the skin of each subject was approximately 1.11 mg. This represents a mean increase of approximately 13 % in the mass of lipstick delivered to the skin per subject for Example 10 over the control (Example Y).

Thus Example 10, which comprises the spray-powder of the invention, exhibits improved topical delivery of lipstick compared to the formulation without the powder.

### Example 11

### Nail enamel with 0.25% spray dried moisture cream

| Ingredient | % |
|---|---|
| Standard nail polish base + 0.25% spray dried moisture cream | 89.65 |
| Calcium fibres | 0.11 |
| Pearl suspension | 0.28 |
| D&C red No 6 dispersion | 6.57 |
| D&C Red No 34 dispersion | 2.07 |
| TiO₂ dispersion | 1.32 |

Calcium fibres were added to a small tub containing the standard nail polish base and the spray dried powder (which was prepared as described in Example B). Then the mixture was stirred until the components were evenly distributed. Pearl suspension was then mixed into this blend until fully integrated. D&C Red No 6 dispersion, D&C Red No 34 dispersion and TiO₂ dispersion were added to the mixture, which was stirred until all the ingredients were evenly dispersed. The material was then bottled and sealed.

### Example 12

### Nail enamel with 0.5% nail spray dried moisture cream

| Ingredient | % |
|---|---|
| Standard nail polish base + 0.5% spray dried powder | 89.68 |
| Calcium fibres | 0.11 |
| Pearl suspension | 0.28 |
| TiO₂ dispersion | 1.31 |
| D&C Red No 34 dispersion | 2.07 |
| D&C Red No 6 dispersion | 6.55 |

Calcium fibres were added to a small tub containing the standard nail polish base and 0.5% spray dried powder (which was prepared as described in Example B), Then the mixture was stirred until the components were evenly distributed. Pearl suspension was then mixed into this blend until fully integrated. TiO₂ dispersion, D&C Red No 34 dispersion and D&C Red No 6 dispersion were added to the mixture, which was stirred until all ingredient were evenly dispersed. The material was then bottled and sealed.

### Example 13

### Nail enamel with 0.75% spray dried moisture cream

| Ingredient | % |
|---|---|
| Standard nail polish base + 0.75% spray dried moisture cream | 89.70 |
| Calcium fibres | 0.11 |
| Pearl suspension | 0.28 |
| D&C Red No 6 dispersion | 6.54 |
| D&C Red No 34 dispersion | 2.06 |
| TiO₂ dispersion | 1.31 |

Calcium fibres were added to a small tub containing the Standard nail polish base and 0.75% spray dried moisture cream (which was prepared as described in Example B), then the mixture was stirred until the components were evenly distributed. Pearl suspension was then mixed into this blend until fully integrated. D&C Red No 6 dispersion, D&C Red No 34 dispersion and TiO₂ dispersion were added to the mixture, which was stirred until all the ingredients were evenly dispersed. The material was then bottled and sealed.

### Example 14

### Lip pencil - softly red (spray dried moisture cream)

| Ingredient | % |
|---|---|
| Microcrystalline wax | 14.56 |
| Camauba wax | 53.36 |
| D&C red No 7 calcium lake | 6.76 |
| Titanium dioxide | 2.51 |
| F D&C yellow No 5 aluminium lake | 7.52 |
| D&C red No 6 barium lake | 4.46 |
| Iron oxides | 1.67 |
| Sorbitan tristearate | 3.00 |
| BHA | 0.03 |
| Spray-dried moisture cream | 0.50 |

The microcrystalline wax and carnauba wax were mixed together and heated to 120°C. D&C red No 7 calcium lake, titanium dioxide, F D&C yellow No 5 aluminium lake, D&C red No 6 barium lake, iron oxides, sorbitan tristearate,BHA and spray-dried moisture cream (which was prepaed as described in Example B) were then dispersed in the oil using a silverson high shear mixer. The mixture was then poured into moulds, then left to stand for a few days to form the hardened pencil lead.

### Example 15

### Eye pencil brown (spray dried moisture cream)

| Ingredient | % |
|---|---|
| Japan wax | 6.75 |
| Hydrogenated palm kernel oil | 13.00 |
| Carnauba wax | 9.62 |
| Hydrogenated coco-glycerides | 27.23 |
| Hydrogenated castor oil | 3.38 |
| Candelilla wax | 6.74 |
| Iron oxides | 30.75 |
| Silica | 0.50 |
| Sorbitan tristearate | 3 |
| BHA | |
| Spray-dried moisture cream | 0.50 |

The Japan wax, hydrogenated palm kernel oil, carnauba was, hydrogenated castor oil and candelilla wax were mixed together and heated to 12°C. Hydrogenated coco-glycerides, iron oxides, silica, sorbitan tristearate, BHA and spray-dried moisture cream were then dispersed in the oil using a silverson high shear mixer. The mixture was then poured into moulds, then left to stand for a few days to form the hardened pencil lead.

## Claims

1. A substantially anhydrous and/or hydrophobic composition in the form of lipsticks, soaps, deodorant sticks, sunscreen sticks, eye pencils, nail enamels or nail lacquers comprising up to 10% by weight of a spray dried powder which is a substantially water-soluble absorbent matrix containing at least one protein obtained from legumes and at least one hydrolysed starch wherein the ratio by weight of protein to hydrolysed starch is in the range 1:20 to 1:2.

2. A composition as claimed in claim 1, in which the ratio by weight of protein to hydrolysed starch is in the range 1:10 to 1:4.

3. A composition as claimed in claim 1, in which the matrix comprises a protein obtained from peas and/or soya beans.

4. A composition as claimed in claim 3, in which the protein is of the type obtained from yellow peas.

5. A composition as claimed in any one of the preceding claims wherein the hydrolysed starch has a molecular weight in the range 1,000 to 100,000.

6. A composition as claimed in any preceding claim, in which the hydrolysed starch is maltodextrin, hydrolysed potato derivative, hydrolysed corn derivative, hydrolysed maize derivative, hydrolysed oat derivative, hydrolysed tapioca derivative, cyclodextrin, dextrin, pullulan and cellulose derivatives.

7. A composition as claimed in any one of the preceding claims in which the hydrolysed starch is maltodextrin having a molecular weight in the range 1,000 to 10,000.

8. A composition as claimed in any preceding claim, which further comprises a surfactant.

9. A composition as claimed in any preceding claim, which further comprises one or more pharmaceutical active ingredients, cosmetic active ingredient, toiletries active ingredient and/or any compatible mixture thereof.

10. A composition as claimed any preceding claim in the form of a lipstick which further comprises a pigment and/or a moisturiser.

11. A composition as claimed in claim 10 which comprises one or more oil components, one or more wax components and/or one or more additional powder components.

12. A composition as claimed in claim 11 which contains 30 to 85% of the oil component, 1 to 40% of the wax component and 1 to 40% of the additional powder component.

13. A composition as claimed in any preceding claim, which is suitable for topical application.

14. A method of preparing a substantially anhydrous and/or hydrophobic composition for non-oral administration as claimed in any preceding claim, comprising the steps of:
(a) spray drying a liquid to form a powder as claimed in any of claims 1 to 13; and
(b) mixing the powder in a suitable manner with other suitable ingredients to form an anhydrous and/or hydrophobic composition suitable for administration by a non-oral route.

## Patentansprüche

1. Im Wesentlichen wasserfreie und/oder hydrophobe Zusammensetzung in der Form von Lippenstiften, Seifen, Deodorantstiften, Sonnenschutzstiften, Augenkonturenstiften, Nagelglasuren oder Nagellacken, umfassend bis zu 10 Gew.-% eines sprühgetrockneten Pulvers, das eine im Wesentlichen wasserlösliche absorptionsfähige Matrix ist, die wenigstens ein von Legumen erhaltenes Protein und wenigstens eine hydrolisierte Stärke enthält, wobei das Gewichtsverhältnis von Protein zu hydrolisierter Stärke im Bereich von 1:20 bis 1:2 liegt.

2. Zusammensetzung nach Anspruch 1, bei dem das Gewichtsverhältnis von Protein zu hydrolisierter Stärke im Bereich von 1:10 bis 1:4 liegt.

3. Zusammensetzung nach Anspruch 1, bei dem die Matrix ein Protein umfasst, das von Erbsen und/oder Sojabohnen erhalten wurde.

4. Zusammensetzung nach Anspruch 3, bei dem das Protein von dem von gelben Erbsen erhaltenen Typ ist.

5. Zusammensetzung nach einem der vorherigen Ansprüche, bei dem die hydrolisierte Stärke eine relative Molekülmasse im Bereich von 1000 bis 100.000 hat.

6. Zusammensetzung nach einem der vorherigen Ansprüche, bei dem die hydrolisierte Stärke Maltrodextrin, hydrolisiertes Kartoffelderivat, hydrolisiertes Getreidederivat, hydrolisiertes Maisderivat, hydrolisiertes Haferderivat, hydrolisiertes Tapiokaderivat, Cyclodextrin, Dextrin, Pullulan- und Cellulosederivate ist.

7. Zusammensetzung nach einem der vorherigen Ansprüche, bei dem die hydrolisierte Stärke Maltrodextrin mit einer relativen Molekülmasse im Bereich von 1000 bis 10.000 ist.

8. Zusammensetzung nach einem der vorherigen Ansprüche, die ferner ein Tensid umfasst.

9. Zusammensetzung nach einem der vorherigen Ansprüche, die ferner ein oder mehrere pharmazeutisch aktive Inhaltsstoffe, einen kosmetisch aktiven Inhaltsstoff, einen Toilettenartikel-aktiven Inhaltsstoff und/oder ein beliebiges kompatibles Gemisch davon umfasst.

10. Zusammensetzung nach einem der vorherigen Ansprüche in der Form eines Lippenstiftes, der ferner ein Pigment und/oder ein Feuchthaltemittel umfasst.

11. Zusammensetzung nach Anspruch 10, die eine oder mehrere Ölkomponenten, eine oder mehrere Wachskomponenten und/oder eine oder mehrere zusätzliche Pulverkomponenten umfasst.

12. Zusammensetzung nach Anspruch 11, die 30 bis 85% der Ölkomponente, 1 bis 40% der Wachskomponente und 1 bis 40% der zusätzlichen Pulverkomponente enthält.

13. Zusammensetzung nach einem der vorherigen Ansprüche, die für eine topische Anwendung geeignet ist.

14. Verfahren zum Herstellen einer im Wesentlichen wasserfreien und/oder hydrophoben Zusammensetzung für die nichtorale Verabreichung nach einem der voherigen Ansprüche, umfassend die folgenden Schritte:
(a) Sprühtrocknen einer Flüssigkeit zur Bildung eines Pulvers nach einem der Ansprüche 1 bis 13; und
(b) Mischen des Pulvers auf geeignete Weise mit anderen geeigneten Inhaltsstoffen zur Bildung einer wasserfreien und/oder hydrophoben Zusammensetzung, die für die Verabreichung auf einem nichtoralen Weg geeignet ist.

## Revendications

1. Composition sensiblement anhydre et/ou hydrophobe présentée sous la forme de bâtonnets de rouge à lèvres, de savons, de bâtonnets déodorants, de bâtonnets de produit écran solaire, de bâtonnets à sourcils, de vernis à ongles ou de laques à ongles comprenant jusqu'à 10% en poids d'une poudre séchée par pulvérisation qui constitue sensiblement une matrice absorbante hydrosoluble contenant au moins une protéine obtenue de légumineux et au moins un amidon hydrolysé, le rapport pondéral entre protéine et amidon hydrolysé étant situé dans la fourchette de 1 :20 à 1 :2.

2. Composition selon la revendication 1, où le rapport pondéral entre protéine et amidon hydrolysé se situe dans la fourchette de 1 :10 à 1 :4.

3. Composition selon la revendication 1, où la matrice comprend une protéine obtenue à partir de pois et/ou de graines de soja.

4. Composition selon la revendication 3, où la protéine est du type obtenu à partir de pois chiches.

5. Composition selon l'une quelconque des revendications précédentes, où l'amidon hydrolysé présente un poids moléculaire situé dans la fourchette de 1 000 à 100 000.

6. Composition selon l'une quelconque des revendications précédentes, où l'amidon hydrolysé est de la malto-dextrine, un dérivé hydrolysé de pomme de terre, un dérivé hydrolysé de blé, un dérivé hydrolysé de maïs, un dérivé hydrolysé d'avoine, un dérivé hydrolysé de tapioca, une cyclo-dextrine, une dextrine, des dérivés de pullulan et de cellulose.

7. Composition selon l'une quelconque des revendications précédentes, où l'amidon hydrolysé est de la malto-dextrine présentant un poids moléculaire situé dans la fourchette de 1 000 à 10 000.

8. Composition selon l'une quelconque des revendications précédentes, comprenant en outre un agent tensio-actif.

9. Composition selon l'une quelconque des revendications précédentes, comprenant en outre une ou plusieurs matières pharmaceutiquement actives, une matière cosmétique active, une matière active de produit de toilette et/ou tout mélange compatible de celles-ci.

10. Composition selon l'une quelconque des revendications précédentes, sous la forme d'un bâtonnet de rouge à lèvres qui contient en outre un pigment et/ou un agent hydratant.

11. Composition selon la revendication 10 qui comprend un ou plusieurs composants huileux, un ou plusieurs composants cireux et/ou un ou plusieurs composants poudreux supplémentaires.

12. Composition selon la revendication 11 qui contient de 30 à 85% du composant huileux, de 1 à 40% du composant cireux et de 1 à 40% du composant poudreux supplémentaire.

13. Composition selon l'une quelconque des revendications précédentes, convenant à une application topique.

14. Procédé de préparation d'une composition sensiblement anhydre et/ou hydrophobe destinée à une administration non orale selon l'une quelconque des revendications précédentes, comprenant les étapes de :
(a) séchage par pulvérisation d'un liquide afin de former une poudre telle que revendiquée dans l'une quelconque des revendications 1 à 13 ; et
(b) mélange de la poudre de façon appropriée avec d'autres ingrédients adéquats pour former une composition anhydre et/ou hydrophobe convenant pour une administration par voie non orale.
